# EUROPEAN PATENT APPLICATION

(11) **EP 4 530 308 A1**
(43) Date of publication of application: **02.04.2025**
(21) Application number: 23932547.5
(22) Date of filing: 21.04.2023
(51) Int. Cl.: C08J 3/24, C08J 3/075, A61K 47/36, A61L 27/20

(54) **MULTI-CROSSLINKED GEL SYSTEM AND PRODUCT THEREOF, PREPARATION METHOD, AND USE**

(30) Priority: 10.04.2023 CN 202310384360
(71) Applicant: Shanghai Qisheng Biological Preparation Co., Ltd., Shanghai 201106 (CN)
(72) Inventor: WEI, Changzheng, Shanghai 201106 (CN); WANG, Xiaotong, Shanghai 201106 (CN); LU, Tingting, Shanghai 201106 (CN); JIANG, Fang, Shanghai 201106 (CN); XI, Hongwei, Shanghai 201106 (CN); JIANG, Lixia, Shanghai 201106 (CN)
(74) Representative: Herrero & Asociados, S.L.
(86) International application number: PCT/CN2023/089767
(87) International publication number: WO 2024/212266

(57) **Abstract**

The present application relates to a multi-crosslinked gel system and a product thereof, a preparation method, and a use. Particularly provided is a preparation method for the multi-crosslinked gel system. The preparation method comprises: under a condition suitable for crosslinking, enabling a polymer compound to undergo a first crosslinking reaction in the presence of a long-chain crosslinking agent to obtain a preliminarily crosslinked gel network; and swelling, precipitating and redissolving the preliminarily crosslinked gel network to form a preliminarily crosslinked gel; under a condition suitable for crosslinking, enabling the preliminarily cross-linked gel to undergo a second crosslinking reaction in the presence of a short-chain crosslinking agent and/or a condensing agent to obtain a secondarily crosslinked gel network; and swelling, precipitating and redissolving the secondarily crosslinked gel network to form a multi-crosslinked gel system. Also provided is a wide use of the multi-crosslinked gel system and the product thereof in medical treatment, beauty, production and daily chemicals

## Description

### TECHNICAL FIELD

The present application belongs to the field of biomedicine and polymer materials. The present application specifically relates to polymer materials that can be used for medical purposes (such as ophthalmology, orthopedics, wound repair, and tissue engineering), plastic surgery, production, or daily use, and more specifically, it relates to a multi-crosslinked gel system and preparation methods and applications thereof.

### BACKGROUND

Hydrogels have great applications in biomedicine and other fields. Hydrogels are usually composed of natural polymers or synthetic polymers, wherein the natural polymers are liable to be recognized by enzymes in the body and therefore be degraded, and synthetic polymers will also be gradually hydrolyzed in the body. Typically, crosslinking is used to increase the stability of polymer hydrogels.

The most common crosslinking method uses a single crosslinking agent to crosslink polymer materials to form a stable three-dimensional network. Since the development of crosslinking technology, its function is not only limited to prolonging the degradation time of hydrogels but also providing effective means to adjust the physical and chemical properties of hydrogels. However, it is difficult for a single crosslinking agent to control the crosslinking conditions to obtain a hydrogel with desired multiple properties, such as stability and cohesion, or toughness and high strength.

In recent years, it has been found that double network or interpenetrating network hydrogels have the advantages of high strength and high toughness. Most of the current double network hydrogels use two-component polymer materials, and the polymers are modified by methacrylamide to form crosslinks under the action of photoinitiators and ultraviolet light (UV-light). However, photoinitiators have certain toxicity, and the safety issues for the human body also remain unknown. Further, the methacrylamide modification process to polymers is also complex.

Other methods include covalent and non-covalent crosslinking. For example, CN 108864494 B discloses a dynamic crosslinked double network hydrogel and preparation methods and applications thereof, wherein the dynamic crosslinked double network hydrogel is formed by interlacing and entanglement of a main network and a secondary network in an aqueous medium. There is no interaction between the main network and the secondary network. The main network is a dynamic covalent bond crosslinked polymer, and the secondary network is an ionic bond crosslinked polymer. However, the ionically crosslinked polymer structure is easily disintegrated in vivo due to the diffusion of ions, and since there is no interaction between the main network and the secondary network, the secondary network cannot be retained for a long time after disintegration, and the overall gel stability is reduced.

Two crosslinking agents can be used to covalently crosslink the double network components to form double covalent bonds, which could increase the stability of the double network. CN 112334168 A discloses a dermal filler comprising double crosslinked collagen and hyaluronic acid and the application thereof, wherein different crosslinking agents have been used to crosslink the two materials. The crosslinking agent is selected from 1,4-butanediol diglycidyl ether (BDDE), 1-[3-(dimethylamino)propyl]-3-ethylcarbodiimide iodide (EDC), N, N'- dicyclohexylcarbodiimide (DCC), N, N'-diisopropylcarbodiimide (DIC), divinyl sulfone (DVS) or glutaraldehyde. However, the above-mentioned crosslinking agents are all short-chain small molecules and do not show other advantages except for increasing stability and viscoelasticity.

There is still a need in the art to develop new polymer materials with high stability, strength, and cohesion, as well as the preparation methods thereof.

### SUMMARY

The present application provides a novel multi-crosslinked gel system and its preparation and application.

In some aspects of the present application, a method for preparing a multi-crosslinked gel system is provided, which comprises the following steps:
(1) subjecting the high-molecular compound to a first crosslinking reaction in the presence of a long-chain crosslinking agent under a condition suitable for crosslinking to obtain a preliminarily crosslinked gel network, wherein the long-chain crosslinking agent has a molecular weight of no less than 400 Da and/or a chain length of no less than 12 backbone skeleton atoms (for example, when the backbone is an alkane chain, the alkane chain comprises 12 or more backbone carbon atoms (≥C12));
(2) swelling (e.g., fully swelling), precipitating, and redissolving the preliminarily crosslinked gel network to form a preliminarily crosslinked gel;
(3) subjecting the preliminarily crosslinked gel to a second crosslinking reaction in the presence of a short-chain crosslinking agent and/or a condensing agent under a condition suitable for crosslinking to obtain a secondary crosslinked gel network, wherein the short-chain crosslinking agent has a molecular weight of less than 400 Da and/or a chain length of less than 12 backbone skeleton atoms (for example, when the backbone is an alkane chain, the alkane chain comprises less than 12 backbone carbon atoms (<C12));
(4) swelling (e.g., fully swelling), precipitating, and redissolving the secondary crosslinked gel network to form a multi-crosslinked gel system.

The method of the present application adopts a combination of a long-chain crosslinking agent and a short-chain crosslinking agent and/or a condensing agent to perform multiple crosslinking on the high-molecular compound. The first layer of loose network is initially formed through long-chain crosslinking, which renders the gel network a certain stability and flexibility and maintains a certain degree of mobility of the polymer. On this basis, the second layer of a dense network is formed through short-chain crosslinking and/or self-crosslinking, which further improves the stability while increasing the rigidity, strength, and cohesion.

In some aspects of the present application, a multi-crosslinked gel system prepared by the method of the present application is provided.

In some aspects of the present application, a preparation comprising the multi-crosslinked gel system prepared by the method of the present application is also provided.

In some aspects of the present application, the use of the multi-crosslinked gel system and preparations of the present application are also provided, which are useful to prepare products for medical treatment, beauty, production, and daily chemicals.

In some aspects of the present application, methods for disease treatment and/or beauty are also provided, which comprise administering to a subject in need thereof a therapeutically or cosmetically effective amount of the multi-crosslinked gel system of the present application or a product or article comprising the multi-crosslinked gel system.

Those skilled in the art may arbitrarily combine the above technical solutions and technical features without departing from the inventive concept and protection scope of the present invention. Other aspects of the present invention are obvious to those skilled in the art based on the disclosure of the present application.

### DESCRIPTION OF DRAWINGS

The present invention will be further illustrated below in conjunction with the accompanying drawings, and these drawings are only for illustrating the embodiments of the present invention rather than limiting the scope of the present invention.
**FIG. 1****:** Scanning electron micrograph of the precipitate before the second redissolution in Example 1.
**FIG. 2****:** Shear viscosity of the double-crosslinked hyaluronic acid gel in Example 1.
**FIG. 3****:** Spectral transmittance of the double-crosslinked hyaluronic acid gel in Example 1.
**FIG. 4****:** Integration of the double-crosslinked hyaluronic acid gel in Example 1 with the pig skin tissue after being coated on the pig dermis and subjected to forces in all directions.
**FIG. 5****:** External observation images at 24h, 48h, 72h, and 7 days after the injection of the double-crosslinked hyaluronic acid gel in Example 2 into the dermis of New Zealand white rabbits.
**FIG. 6****:** Observation image of the gel and tissue inside the dermis at 7 days after the injection of the double-crosslinked hyaluronic acid gel in Example 2 into the dermis of New Zealand white rabbits.
**FIG. 7****:** The Hematoxylin-eosin (HE) staining image of the gel and skin tissues at 7 days after the injection of the double-crosslinked hyaluronic acid gel in Example 2 into the dermis of New Zealand white rabbits.
**FIG. 8****:** Gross observation image of the eyeball at 16 weeks after injection of the double-crosslinked hyaluronic acid gel in Example 3 into the vitreous cavity of New Zealand white rabbits.
**FIG. 9****:** Image of the fundus oculi at 16 weeks after injection of the double-crosslinked hyaluronic acid gel in Example 3 into the vitreous cavity of New Zealand white rabbits.
**FIG. 10****:** Intraocular pressure (IOP) measurements within 16 weeks after injection of the double crosslinked hyaluronic acid gel in Example 3 into the vitreous cavity of New Zealand white rabbits.
**FIG. 11****:** HE staining image of the retina at 16 weeks after injection of the double-crosslinked hyaluronic acid gel in Example 3 into the vitreous cavity of New Zealand white rabbits.
**FIG. 12****:** Scanning electron microscopy image of the double-crosslinked carboxymethyl chitosan gel in Example 4.
**FIG. 13****:** Live-dead staining image of the double-crosslinked hyaluronic acid-collagen gel in Example 5 after co-culture with fibroblasts for 7 days.
**FIG. 14****:** Photo of the double-crosslinked carboxymethyl chitosan gel-microsphere composite in Example 6.
**FIG. 15****:** Scanning electron microscopy image of the double-crosslinked carboxymethyl chitosan gel-microsphere composite in Example 6.
**FIG. 16****:** Microscope image of the double-crosslinked carboxymethyl chitosan gel-microsphere composite in Example 7 after co-culture with fibroblasts for 7 days.
**FIG. 17****:** Masson's trichrome staining image of the tissue at one month after the implantation of the double-crosslinked carboxymethyl chitosan gel-microsphere composite in Example 8 into the dermis of New Zealand white rabbits.
**FIG. 18****:** Sirius red staining image of the tissue at one month after implantation of the double-crosslinked carboxymethyl chitosan gel-microsphere composite in Example 8 into the dermis of New Zealand white rabbits.
**FIG. 19****:** Scanning electron microscope image of the precipitate obtained without applying gradient temperature increase during the poor solvent replacement process in Comparative Example 1.

### DETAILED DESCRIPTION

In previous crosslinking technologies, the effect of the length of the crosslinking agent molecular chain on gel properties was overlooked. Through long-term and in-depth research, the inventor unexpectedly found that the length of the crosslinking agent molecular chain affects the spatial structure and mobility of the polymer, which in turn has a significant impact on gel properties. Combining long-chain and short-chain crosslinking agents to double crosslink the high-molecular compound can provide the resulting multi-crosslinked gel system with enhanced performance.

In the present application, the introduction of long-chain crosslink(s), short-chain crosslink(s), and/or self-crosslink(s) into the polymer network forms a multi-crosslinked network that improves the stability and strength of the gel and simultaneously provides gel flexibility and cohesion. The gel systems, preparations, and products provided in the present application can be used in the fields of soft tissue repair, vitreous filling, and wound repair, and can also be coated with cells, growth factors, and microspheres, allowing for extensive use in fields such as biomedicine, cosmetic surgery, tissue engineering, and daily chemicals.

For example, hyaluronic acid and its derivatives, such as sodium hyaluronate, have been widely tested in the human body as vitreous substitutes since the 1960s due to their good biocompatibility and tolerance. However, since hyaluronic acid and its derivatives degrade quickly in the body and have a short residence time, they are not suitable for use as long-term fillers. Furthermore, their limitations in rheological and mechanical properties constrain their applications. The gel obtained through the multi-crosslinking of hyaluronic acid materials using the method described herein exhibits significantly improved properties, such as enhanced stability, strength, and cohesion, making it more suitable for use as a vitreous substitute.

All numerical ranges provided herein are intended to include all values falling between the range endpoints and the numerical ranges therebetween. Features mentioned in the present application or the embodiments may be combined. All features disclosed in the present application may be used in combination with any composition form, and each feature disclosed in the present application may be replaced with any alternative feature that provides the same, equivalent, or similar purpose. Unless otherwise specified, the disclosed features are only general examples of equivalent or similar features.

As used herein, "containing", "having", or "including" encompasses "comprising", "consisting mainly of" "consisting essentially of", and "consisting of". The terms "consisting mainly of", "consisting essentially of", and "consisting of" are subordinate concepts of "containing", "having", or "including".

### Preparation method of multi-crosslinked gel system

The present application provides a method for preparing a multi-crosslinked gel system, which comprises the following steps:
(1) subjecting the high-molecular compound to a first crosslinking reaction in the presence of a long-chain crosslinking agent under a condition suitable for crosslinking to obtain a preliminarily crosslinked gel network, wherein the long-chain crosslinking agent has a molecular weight of no less than 400 Da and/or has a chain length of no less than 12 backbone skeleton atoms (for example, when the backbone is an alkane chain, the alkane chain comprises 12 or more backbone carbon atoms (≥C12));
(2) swelling (preferably, fully swelling), precipitating, and redissolving the preliminarily crosslinked gel network to form a preliminarily crosslinked gel;
(3) subjecting the preliminarily crosslinked gel to a second crosslinking reaction in the presence of a short-chain crosslinking agent and/or a condensing agent under a condition suitable for crosslinking to obtain a secondary crosslinked gel network, wherein the short-chain crosslinking agent has a molecular weight of less than 400 Da and/or a chain length of less than 12 backbone skeleton atoms (for example, when the backbone is an alkane chain, the alkane chain comprises less than 12 backbone carbon atoms (<C12));
(4) swelling, precipitating, and redissolving the secondary crosslinked gel network to form a multi-crosslinked gel system.

In some embodiments, the molecular weight of the polymer compound is no less than 10,000 Da. In some embodiments, the molecular chain of the high-molecular compound is no less than 100 kDa, 300 kDa, 500 kDa, 800 kDa, 1000 kDa, 1500 kDa, 1800 kDa, 2000 kDa, or any sub-range or numerical point therein. In some embodiments, the side chain of the high-molecular compound comprises two or more cross-linkable groups, such as carboxyl, amino, hydroxyl, or a combination thereof.

The high-molecular compounds that can be used in the present application include but are not limited to, one or more selected from the group consisting of hyaluronic acid (such as sodium hyaluronate), chitosan, carboxymethyl chitosan, chitin, carboxymethyl chitin, collagen, gelatin, silk fibroin, carboxymethyl dextran, carboxymethyl cellulose, amino polyethylene glycol, and carboxyl polyethylene glycol.

In some embodiments, a single high-molecular compound can be used as a starting material. In some embodiments, a combination of two or more high-molecular compounds can also be used, such as a combination of hyaluronic acid and collagen. In the combination of two or more high-molecular compounds, the components can be bonded by non-covalent bonds such as covalent crosslinking bonds, hydrogen bonds, and electrostatic attraction.

In some embodiments, the high-molecular compound is dissolved in an aqueous solution, for example, to have an initial concentration of 0.2 wt % to 10 wt %, such as 0.4 wt % to 8 wt %. The initial concentration of the high-molecular compound can be adjusted as needed.

In some embodiments, the molecular weight of the long-chain crosslinking agent that can be used in the present method is no less than 400 Da, for example, no less than 0.5 kDa, 1 kDa, 10 kDa, 50 kDa, 100 kDa, 300 kDa, 500 kDa, 600 kDa, 1000 kDa, 1500 kDa, 1800 kDa, 2000 kDa, or any sub-range or numerical point therein.

In some embodiments, the long chain crosslinking agent that can be used in the present method has a chain length of no less than 12 backbone skeleton atoms, for example, a chain length of no less than 12, 14, 16, 18, 20, 22, 24, 26, 28, 30 backbone skeleton atoms. In some embodiments, the backbone of the long chain crosslinking agent may be a saturated or unsaturated straight chain, branched chain, or cyclic hydrocarbon chain. In some embodiments, in addition to carbon atoms, the backbone of the long-chain crosslinking agent may comprise one or more heteroatoms selected from N, O, and S. In some embodiments, the backbone of the long chain crosslinking agent may comprise atoms other than C, N, O, and S, such as atoms such as silicon, aluminum, titanium, boron, *etc.* In some embodiments, the backbone of the long-chain crosslinking agent may comprise a cyclic portion.

In some embodiments, the long-chain crosslinking agents that can be used in the present method include but are not limited to, the long-chain crosslinking agent(s) selected from the group consisting of amino polyethylene glycol, carboxyl polyethylene glycol, polyethylene glycol diglycidyl ether (PEGDE), polyglutamic acid, polylysine, polyaspartic acid and salts thereof, tannic acid, and proanthocyanidins, with a molecular weight of no less than 400 Da and/or a chain length of no less than 12 backbone skeleton atoms.

In some embodiments, the molar ratio of the long-chain crosslinking agent to the crosslinking reactive group(s) in the high-molecular compound is 1:10 to 1:2.

In some embodiments, the molecular weight of the short chain crosslinking agent that can be used in the present method is less than 400 Da, such as less than 300 Da, 200 Da, 150 Da, 100 Da, 80 Da, 50 Da, or any sub- range or value point therein.

In some embodiments, the short chain crosslinking agent that can be used in the present method has a chain length of less than 12 backbone skeleton atoms, for example, a chain length of less than 12, 11, 10, 9, 8, 7, 6, 5, 4, 3, 2 backbone skeleton atoms. In some embodiments, the backbone of the short chain crosslinking agent may be a saturated or unsaturated straight chain, branched chain, or cyclic hydrocarbon chain. In some embodiments, in addition to carbon atoms, the backbone of the short-chain crosslinking agent may comprise one or more heteroatoms selected from N, O, and S. In some embodiments, the backbone of the short chain crosslinking agent may comprise atoms other than C, N, O, and S, such as atoms such as silicon, aluminum, titanium, boron, *etc.* In some embodiments, the backbone of the short-chain crosslinking agent may comprise a cyclic portion.

In some embodiments, the short-chain crosslinking agents that can be used in the present method include but are not limited to, the short-chain crosslinking agent(s) selected from the group consisting of glutamic acid, lysine, aspartic acid, and salts and polymers thereof, glutamine, divinyl sulfone (DVS), butanediol diglycidyl ether (BDDE), polyethylene glycol diglycidyl ether (PEGDE), diepoxyoctane (DEO), cystamine and its salts, hexamethylenediamine, p-phenylene carbodiimide (BCDI), aminopolyethylene glycol, carboxylpolyethylene glycol, glutaraldehyde, and genipin, with a molecular weight of less than 400 Da and/or a chain length of less than 12 backbone skeleton atoms.

In some embodiments, the molar ratio of the short-chain crosslinking agent to the crosslinking reactive group(s) in the high-molecular compound is 1:10 to 1:1.

In some embodiments, the molecular weight difference between the long chain crosslinking agent and the short chain crosslinking agent is 1 to 10 orders of magnitude, for example, 1, 2, 3, 4, 5, 6, 7, 8, 9, or 10 orders of magnitude. In some embodiments, the molecular weight difference between the long-chain crosslinking agent and the short-chain crosslinking agent is greater than 0.5 kDa, 1 kDa, 10 kDa, 50 kDa, 100 kDa, 500 kDa, 1000 kDa, 1500 kDa, or 2000 kDa.

In some embodiments, the number of backbone skeleton atoms of the long chain crosslinking agent and the short chain crosslinking agent differs by 1 to 3 orders of magnitude, for example, by 1, 2, or 3 orders of magnitude. In some embodiments, the number of backbone skeleton atoms of the long chain crosslinking agent and the short chain crosslinking agent differs by more than 2, 4, 6, 8, 10, 15, 20, 25, 30, 40, 50, 60, 70, 80, 90, 100, 150, 200, 300, 500.

In some embodiments, the crosslinking agent can be independently selected from a homobifunctional crosslinking agent, a heterobifunctional crosslinking agent, or a photoreactive crosslinking agent. In some embodiments, the reactive end of the crosslinking agent can comprise an amine, carboxyl, thiol, succinimidyl, imidoester, or other active groups.

In some embodiments, optionally, step (1) and/or step (3) may further comprise adding a condensing agent to the crosslinking reaction system. When the high-molecular compound has paired reactive group members (e.g., carboxyl and amino reactive groups) in its molecular chain, the condensing agent in the crosslinking reaction system causes the molecules to self-crosslink.

In some embodiments, the condensing agent that can be used in the present method includes but is not limited to a condensing agent selected from the group consisting of 1-ethyl-3-(3-dimethyl aminopropyl)carbodiimide hydrochloride (EDC), 1,3-dicyclohexylcarbodiimide (DCC), glutamine aminotransferase, 4-(4,6-dimethoxytriazine-2-yl)-4-methyl morpholine hydrochloride (DMTMM), 6-chloro-2,4-dimethoxy-1,3,5-triazine (CDMT).

In some embodiments, the molar ratio of the reactive groups of the condensing agent to the crosslinking agent in the reaction system comprising the condensing agent is 1:5 to 2:1.

In some embodiments, step (1) further comprises providing an aqueous solution of the high-molecular compound and adjusting its pH to be suitable for the first crosslinking reaction. In some embodiments, the aqueous solution is selected from phosphate buffer solution, acetic acid solution, or aqueous solution. In some embodiments, the mass percentage concentration of the high-molecular compound in the solution is 0.4% to 8%. In some embodiments, the reaction temperature of the first crosslinking reaction is 0°C to room temperature (RT), for example, 2°C to 30°C, 5°C to 25°C. In some embodiments, the reaction time of the first crosslinking reaction is 5 to 120 hours, for example, 8 to 100 hours, 12 to 72 hours, and 24 to 48 hours. The above conditions can be adjusted as needed.

In some embodiments, step (2) and step (4) each independently comprise: (a) adjusting the pH of the gel network obtained in the previous step(s), for example: when the high-molecular compound with the highest concentration in the system is hyaluronic acid, silk fibroin, carboxymethyl dextran, carboxymethyl cellulose, carboxy polyethylene glycol, the pH value is adjusted to 7.5-14; when the high-molecular compound with the highest concentration in the system is chitosan, carboxymethyl chitosan, chitin, carboxymethyl chitin, collagen, gelatin, amino polyethylene glycol, the pH value is adjusted to 2-6.5. In some embodiments, step (2) and step (4) each independently comprise (b) subjecting the gel network to fully swell and balance in an aqueous solvent. By adjusting the pH and fully swelling the long-chain crosslinked network, the long-chain crosslinked network can be fully stretched and the uncrosslinked groups can be exposed, providing sufficient space for the short-chain crosslinking in the next step.

In some embodiments, step (2) and step (4) each independently comprise adding a poor solvent to an aqueous solution containing a fully swollen gel network and mixing to precipitate the gel network. In some embodiments, the mixture is subjected to gradient heating during the precipitation process, for example, gradually increasing the temperature from 0°C to 50°C, such as 2 to 5°C for 0.5 to 2 hours, 15 to 25°C for 0.5 to 2 hours, and 35 to 45°C for 1 to 4 hours. In some embodiments, the poor solvent is selected from acetone, ethanol, chloroform, ether, n-hexane, or a combination thereof. After mixing uniformly in these steps, the gradient temperature increase is performed. First, the poor solvent is fully mixed with the polymer aqueous phase solution at a relatively low temperature to relatively completely replace the polymer precipitate, and then the temperature is gradually increased to volatilize the poor solvent remaining in the polymer precipitate, so that the polymer precipitate forms a loose and porous structure, avoiding polymer agglomeration and facilitating subsequent redissolution.

In some embodiments, the obtained precipitate is washed once or multiple times. In some embodiments, the precipitate is further redissolved in an aqueous solution, for example, to obtain a mass percentage concentration of 1 to 10% (such as 1.5-8%) gel solution.

In some embodiments, step (3) comprises adjusting the pH of the preliminary crosslinked gel obtained in the previous step. In some embodiments, step (3) comprises performing a gradient dilution during the crosslinking reaction, for example, performing a 1-5-fold dilution after 8-72 hours of reaction; performing a 1-5-fold dilution after continuing the reaction for 8-48 hours, and continuing the reaction for 8-24 hours.

In some embodiments, since the polymer crosslinking process involves the short-chain crosslinking agent(s), if the polymer concentration is high, the reaction will be too fast, leading to brittleness, while if the polymer concentration is low, the reaction efficiency will be very low. Therefore, partial short-chain crosslinking is performed when the polymer concentration is high, and then gradual dilution is carried out. In this way, the reaction rate is controlled, making the reaction easier to manage. Moreover, each step of dilution facilitates the expansion of the crosslinking network, exposes uncrosslinked groups, and improves the crosslinking efficiency.

In some embodiments, the reaction temperature of the second crosslinking reaction is 0°C to RT, such as 2°C to 30°C. In some embodiments, the reaction time of the second crosslinking reaction is 5 to 120 hours, such as 8 to 100 hours.

In some embodiments, the method further comprises performing post-treatment such as purification and/or sterilization on the obtained multi-crosslinked gel system. In some embodiments, the modification degree of the long-chain crosslinking agent in the obtained multi-crosslinked gel system is 2% to 40%, and the modification degree of the short-chain crosslinking agent or self-crosslinking agent is 5% to 40%.

In some exemplary embodiments, the method may comprise the following steps:
(1') preparing an aqueous solution of a high-molecular compound, adjusting the pH, adding a long-chain crosslinking agent, and performing a first crosslinking reaction with or without the presence of a condensing agent to obtain a preliminarily crosslinked gel network;
(2') adjusting the pH, and then placing the gel network from step (1') in an aqueous solution to fully swell and balance, adding a poor solvent, mixing uniformly and then gradually increasing the temperature, collecting the precipitate and repeatedly washing it, redissolving it in an aqueous solution, and formulating a preliminary crosslinked gel with a certain concentration;
(3') adjusting the pH, adding a short-chain crosslinking agent and/or a condensing agent to the preliminary crosslinked gel to carry out a second crosslinking reaction, and performing a gradient dilution during the reaction;
(4') taking the gel obtained from step (3'), adjusting the pH, and then placing the gel network in step (3') in an aqueous solution to fully swell and balance, adding a poor solvent, mixing uniformly, and then gradually heating, collecting the precipitate and repeatedly washing it, and redissolving it in an aqueous solution to obtain a multi-crosslinked gel network;
(5') optionally, the multi-crosslinked gel network is purified, sterilized, and/or packaged.

In some embodiments, the monomer in the multi-crosslinked gel system is hyaluronic acid or the salt thereof, the long-chain crosslinking agent is polyethylene glycol diacrylate (PEGDA), and the short-chain crosslinking agent is divinyl sulfone (DVS). In some embodiments, the monomer in the multi-crosslinked gel system is carboxymethyl chitosan (CMCS), the long-chain crosslinking agent is polyglutamic acid (e.g., with a molecular weight of 1kDa to 100kDa), and the short chain crosslinking agent is cystamine. In some embodiments, the monomer in the multi-crosslinked gel system is collagen, the long-chain crosslinking agent is polylysine (e.g., with a molecular weight of 1kDa to 100kDa), and the short-chain crosslinking agent is glutamine. In some embodiments, the multi-crosslinked gel system is further complexed with microspheres, and the microspheres may be, for example, hydroxyapatite microspheres.

### Multi-crosslinked gel system

Also provided herein is a multi-crosslinked gel system, wherein the multi-crosslinked gel system is prepared using the method described herein.

In some embodiments, the multi-crosslinked gel system described herein comprises a multi-high-molecular compound network structure obtained by crosslinking a long-chain crosslinking agent, crosslinking a short-chain crosslinking agent, or self-crosslinking. In some embodiments, the modification degree of the long-chain crosslinking agent in the multi-crosslinked gel system is 2% to 40%, and the modification degree of the short-chain crosslinking agent or self-crosslinking is 5% to 40%.

In some embodiments, the source of the high-molecular compound part in the multi-crosslinked gel system described herein includes but is not limited to, one or more selected from the group consisting of hyaluronic acid (such as sodium hyaluronate), chitosan, carboxymethyl chitosan, chitin, carboxymethyl chitin, collagen, gelatin, silk protein, carboxymethyl dextran, carboxymethyl cellulose, amino polyethylene glycol, and carboxyl polyethylene glycol. In some embodiments, the multi-crosslinked gel system described herein comprises a single high-molecular compound part. In some embodiments, the multi-crosslinked gel system described herein comprises a part derived from two or more high-molecular compounds, such as a combination of hyaluronic acid and collagen. In the combination of two or more high-molecular compounds, the components can be bonded by non-covalent bonds such as covalent crosslinking bonds, hydrogen bonds, and electrostatic attraction.

In some embodiments, the source of the long-chain crosslinked parts in the multi-crosslinked gel system of the present application includes but is not limited to, one or more long-chain crosslinking agents selected from the group consisting of amino polyethylene glycol, carboxyl polyethylene glycol, polyethylene glycol diglycidyl ether (PEGDE), polyglutamic acid, polylysine, polyaspartic acid and salts thereof, tannic acid, and proanthocyanidins.

In some embodiments, the source of the short-chain crosslinked moieties in the multi-crosslinked gel system of the present application includes but is not limited to, one or more short-chain crosslinking agents selected from the group consisting of glutamic acid, lysine, aspartic acid and salts and polymers thereof, glutamine, divinyl sulfone (DVS), butanediol diglycidyl ether (BDDE), polyethylene glycol diglycidyl ether (PEGDE), diepoxyoctane (DEO), cystamine and salts thereof, hexamethylenediamine, para-phenylene carbodiimide (BCDI), amino polyethylene glycol, carboxyl polyethylene glycol, glutaraldehyde, and genipin.

The multi-crosslinked gel system described herein combines long-chain crosslinking agents with short-chain crosslinking agents or self-crosslinking, wherein it has a first-layer loose network of long-chain crosslinking, conferring the gel network with certain stability and flexibility, and maintaining a certain degree of mobility of the polymer; it also has a second-layer dense network formed by short-chain crosslinking or self-crosslinking, which further improves stability while increasing rigidity and strength. Through the regulation of the length and ratio of the crosslinking agent molecular chain, the multi-crosslinked gel system described herein can control the strength, flexibility, and cohesion of the gel, while improving the gel's resistance to degradation and prolonging its retention time in the body.

As used herein, "cohesion" can be measured as follows (for example, see: Dermatol Surg 2015; 41 Suppl 1: S365-372): The sample is loaded into a 1 mL glass syringe, and the air in the gel is removed through centrifugation at 2500 ~ 3000 rpm for 5 ~ 10 minutes. An 18G needle (inner diameter of 0.9 mm) and a plunger are configured on the syringe. The gel is extruded at a constant speed of 7.5 mm/min using a tensile testing machine. When the displayed force value is stable, the last 10 drops of extruded gel are collected and weighed, and the average mass of each drop of gel is calculated. As used herein, "good cohesion" means that when tested by the above method, the average mass of each drop of gel is no less than 40 mg. The multi-crosslinked gel system of the present application has good cohesion, that is, when tested by the above method, the average mass of each drop of gel is no less than 40 mg, for example, no less than 45, 50, 55, 60, 65, 70, 80, 90, or 100 mg, or any numerical point or numerical range therein.

### Products and Applications

Regarding the excellent performance of the multi-crosslinked gel system of the present application, the material has a wide range of application prospects. Therefore, the present application also provides products comprising the multi-crosslinked gel system of the present invention and applications thereof.

In some embodiments, the multi-crosslinked gel and preparation described herein can be used to prepare products for medical treatment, beauty, production, and daily chemicals. In some embodiments, provided herein are products comprising a multi-crosslinked gel system, the products including but not limited to products selected from the group consisting of medical supplies, beauty supplies, production supplies, and daily chemicals. In some embodiments, the product is selected from the group consisting of vitreous fillers or substitutes, soft tissue or cartilage repairs or fillers, plastic fillers, wound dressings or repairs, drug (e.g., chemical drugs, biological drugs) carriers or drug delivery systems, cell (e.g., stem cells, immune cells) growth or culture carriers or systems, tissue engineering products, and personal care products.

In some embodiments, the product is or comprises a multi-crosslinked gel-microsphere composite, wherein the microsphere includes one or more selected from the group consisting of hydroxyapatite microsphere, polycaprolactone microsphere, polylactic acid microsphere, polyglycolide-lactide microsphere, polyhydroxyalkanoate microsphere, hyaluronic acid microsphere, chitosan microsphere, collagen microsphere, gelatin microsphere, liposome nano-microsphere, and fluorescent microsphere. In some embodiments, the product is or comprises a multi-crosslinked gel system loaded with cells and/or drugs and/or growth factors, wherein the cells and/or drugs and/or growth factors are directly encapsulated in the gel system or are encapsulated in a microsphere and then uniformly mixed with the gel system. For example, the microsphere includes one or more selected from the group consisting of hydroxyapatite microsphere, polycaprolactone microsphere, polylactic acid microsphere, polyglycolide-lactide microsphere, polyhydroxyalkanoate microsphere, hyaluronic acid microsphere, chitosan microsphere, collagen microsphere, gelatin microsphere, liposome nano-microsphere, and fluorescent microsphere.

The multi-crosslinked gel system and the product thereof described herein have good cohesion and stability and are not easy to diffuse when used for soft tissue filling and exert long-term filling effects. Due to the good cohesion, cells or microspheres can be coated so that they do not shift or fall apart in the human body and can be filled in the desired parts for a long time. The gel system can also be used to coat growth factors, therefore having a long-term sustained release effect.

The multi-crosslinked gel system and products thereof described herein have a very low swelling rate when used for vitreous filling, and will not cause increased intraocular pressure; degrade slowly and remain in the body for a long time, allowing a long-term vitreous filling; and do not disintegrate into small particles after degradation and do not block the intraocular angle.

Accordingly, the present application also provides the use of the multi-crosslinked gel system described herein in disease treatment or cosmetic surgery and a method for disease treatment or cosmetic surgery. The method comprises administering a therapeutically effective amount or a cosmetically effective amount of the multi-crosslinked gel system or product described herein to a subject in need thereof.

### Exemplary Embodiments

The following exemplary embodiments are provided in the present application. It should be understood that after reading the contents of this application, those skilled in the art may conduct various changes or modifications to the present application, and these equivalent forms also fall within the scope defined by the claims attached to this application.

In some aspects of the present application, a method for preparing a multi-crosslinked gel system is provided, which comprises the following steps:
(1) subjecting the high-molecular compound to a first crosslinking reaction in the presence of a long-chain crosslinking agent under a condition suitable for crosslinking to obtain a preliminarily crosslinked gel network, wherein the long-chain crosslinking agent has a molecular weight of no less than 400 Da and/or has a chain length of no less than 12 backbone skeleton atoms;
(2) swelling, precipitating, and redissolving the preliminarily crosslinked gel network to form a preliminarily crosslinked gel;
(3) subjecting the preliminarily crosslinked gel to a second crosslinking reaction in the presence of a short-chain crosslinking agent and/or a condensing agent under a condition suitable for crosslinking to obtain a secondary crosslinked gel network, wherein the short-chain crosslinking agent has a molecular weight of less than 400 Da and/or a chain length of less than 12 backbone skeleton atoms;
(4) swelling, precipitating, and redissolving the secondary crosslinked gel network to form a multi-crosslinked gel system.

In some embodiments, the molecular weight of the high-molecular compound used is no less than 10,000 Da, and the side chain comprises two or more carboxyl groups, amino groups, hydroxyl groups, or a combination thereof.

In some embodiments, the high-molecular compound used is one or more selected from the group consisting of hyaluronic acid (such as sodium hyaluronate), chitosan, carboxymethyl chitosan, chitin, carboxymethyl chitin, collagen, gelatin, silk fibroin, carboxymethyl dextran, carboxymethyl cellulose, amino polyethylene glycol, and carboxyl polyethylene glycol.

In some embodiments, the initial concentration of the high-molecular compound in the aqueous solution is 0.2% to 10%, such as 0.4% to 8%.

In some embodiments, the long-chain crosslinking agent used is selected from the group consisting of amino polyethylene glycol, carboxyl polyethylene glycol, polyethylene glycol diglycidyl ether (PEGDE), polyglutamic acid, polylysine, polyaspartic acid and their salts, tannic acid, proanthocyanidins, and has a molecular weight of no less than 400 Da and/or a chain length of no less than 12 backbone skeleton atoms. In some embodiments, the molar ratio of the long-chain crosslinking agent used to the crosslinking reactive group(s) in the high-molecular compound is 1:10 to 1:2.

In some embodiments, the short chain crosslinking agent used is selected from the group consisting of glutamic acid, lysine, aspartic acid, and their salts and polymers, glutamine, divinyl sulfone (DVS), butanediol diglycidyl ether (BDDE), polyethylene glycol diglycidyl ether (PEGDE), diepoxyoctane (DEO), cystamine and salts thereof, hexamethylenediamine, p-phenylene carbodiimide (BCDI), amino polyethylene glycol, carboxylpolyethylene glycol, glutaraldehyde, genipin, and has a molecular weight of less than 400 Da and/or a chain length of less than 12 backbone skeleton atoms. In some embodiments, the molar ratio of the short-chain crosslinking agent used to the crosslinking reactive group(s) in the high-molecular compound is 1:10 to 1:1.

In some embodiments, optionally, step (1) and/or step (3) may also comprise adding a condensing agent to the crosslinking reaction system. In some embodiments, the condensing agent used is selected from the group consisting of 1-ethyl-3-(3-dimethylaminopropyl) carbodiimide hydrochloride (EDC), 1,3-dicyclohexylcarbodiimide (DCC), glutamine aminotransferase, 4-(4,6-dimethoxytriazine-2-yl)-4-methylmorpholine hydrochloride (DMTMM), 6-chloro-2,4-dimethoxy-1,3,5-triazine (CDMT). In some embodiments, the molar ratio of the reactive groups of the condensing agent to the crosslinking agent in the reaction system comprising the condensing agent is 1:5 to 2:1. In some embodiments, when the molecular chain of the high-molecular compound used has both carboxyl and amino reactive groups, the condensing agent in the crosslinking reaction system causes the molecules to self-crosslink.

In some embodiments, step (1) further comprises providing an aqueous solution of the high-molecular compound (e.g., a phosphate buffer solution, or an aqueous solution), and adjusting its pH to be suitable for the first crosslinking reaction. In some embodiments, the aqueous solution used is selected from a phosphate buffer solution, an acetic acid solution, or an aqueous solution. In some embodiments, the mass percentage concentration of the high-molecular compound used in the solution is 0.4% to 8%. In some embodiments, the reaction temperature of the first crosslinking reaction is 0°C to RT, for example, 2°C to 30 °C. In some embodiments, the reaction time of the first crosslinking reaction is 5 to 120 hours, for example, 8 to 100 hours.

In some embodiments, step (2) and step (4) may each optionally and independently comprise one or more selected from the group consisting of:
(a) adjusting the pH of the gel network obtained in the previous step, for example:
   When the high-molecular compound with the highest concentration in the system is hyaluronic acid, silk fibroin, carboxymethyl dextran, carboxymethyl cellulose, and/or carboxy polyethylene glycol, the pH value is adjusted to 7.5 to 14;
   When the high-molecular compound with the highest concentration in the system is chitosan, carboxymethyl chitosan, chitin, carboxymethyl chitin, collagen, gelatin, and/or amino polyethylene glycol, the pH value is adjusted to 2 to 6.5;
(b) the swelling comprises: fully swelling and equilibrating the obtained gel network in an aqueous solution;
(c) the precipitating comprises: adding a poor solvent to an aqueous solution comprising the fully swollen gel network and mixing to precipitate the gel network, for example:
   gradually heating the mixture during the precipitation process, for example, from 0°C to 50°C, such as 2 to 5°C for 0.5 to 2 hours, 15 to 25°C for 0.5 to 2 hours, and 35 to 45°C for 1 to 4 hours;
   the poor solvent is selected from acetone, ethanol, chloroform, ether, n-hexane, or a combination thereof;
(d) optionally, washing the resulting precipitate one or more times; and/or
(e) the re-dissolving comprises: re-dissolving part or all of the precipitate in an aqueous solution, for example, to obtain a mass percentage concentration of 1 to 10% (such as 1.5-8%) gel solution.

In some embodiments, the method of the present application comprises performing gradient heating during the precipitation of the gel network with a poor solvent. In some embodiments, the gradient heating is gradually increased from 0°C to 50°C, such as 2 to 5°C for 0.5 to 2 hours, 15 to 25°C for 0.5 to 2 hours, and 35 to 45°C for 1 to 4 hours.

In some embodiments, step (3) comprises adjusting the pH of the preliminary crosslinked gel obtained in the previous step. In some embodiments, step (3) comprises performing a gradient dilution during the crosslinking reaction. In some embodiments, the gradient dilution is, for example, 1 to 5 times dilution after 8 to 72 hours of reaction, followed by 1 to 5 times dilution after 8 to 48 hours of reaction, and then 8 to 24 hours of reaction.

In some embodiments, the reaction temperature of the second crosslinking reaction is 0°C to RT, for example, 2°C to 30°C. In some embodiments, the reaction time of the second crosslinking reaction is 5 to 120 hours, for example, 8 to 100 hours.

In some embodiments, the preparation method of the multi-crosslinked gel system described herein further comprises purifying and/or sterilizing the obtained multi-crosslinked gel system. In some embodiments, the modification degree of the long-chain crosslinking agent in the multi-crosslinked gel system described herein is 2% to 40%, and the modification degree of the short-chain crosslinking agent or self-crosslinking agent is 5% to 40%.

In some embodiments, the preparation of the multi-crosslinked gel system herein comprises the following steps:
(1') preparing an aqueous solution of a high-molecular compound, adjusting the pH, adding a long-chain crosslinking agent, and performing a first crosslinking reaction with or without the presence of a condensing agent to obtain a preliminarily crosslinked gel network;
(2') adjusting the pH, and then placing the gel network in step (1') in an aqueous solution to fully swell and balance, adding a poor solvent, mixing uniformly and then gradually increasing the temperature, taking the precipitate and repeatedly washing, redissolving it in an aqueous solution, and formulating a preliminary crosslinked gel with a certain concentration;
(3') adjusting the pH, adding a short-chain crosslinking agent and/or a condensing agent to the preliminary crosslinked gel to carry out a second crosslinking reaction, and performing a gradient dilution during the reaction;
(4') taking the gel obtained in step (3'), adjusting the pH, and then placing the gel network in step (3') in an aqueous solution to fully swell and balance, adding a poor solvent, mixing uniformly, and then gradually heating, taking the precipitate and repeatedly washing, and redissolving it in an aqueous solution to obtain a multi-crosslinked gel network;
(5') optionally, purifying, sterilizing, and/or packaging the multi-crosslinked gel network.

In some embodiments, the method for preparing the multi-crosslinked gel system described herein further comprises selecting the lengths of the long-chain crosslinking agent and the short-chain crosslinking agent and/or the dosage ratio of the long-chain crosslinking agent to the short-chain crosslinking agent to control the crosslinking modification degree of the multi-crosslinked gel network.

In some aspects of the present application, a multi-crosslinked gel system is provided, wherein the multi-crosslinked gel system is prepared by the method according to the present application.

In some aspects of the present application, a multi-crosslinked gel system is provided, wherein the multi-crosslinked gel system comprises a first layer network obtained by crosslinking using a long-chain crosslinking agent; and a second layer network obtained by using a short-chain crosslinking agent or self-crosslinking-,-wherein the density of the first layer network is lower than that of the second layer network.

In some embodiments, the multi-crosslinked gel system described herein has an average cohesion of no less than 40 mg per drop of gel as measured by loading the sample into a 1 mL glass syringe, and centrifuging at 2500-3000 rpm for 5-10 minutes to remove the air in the gel; installing an 18G needle with an inner diameter of 0.9 mm and a plunger on the syringe; extruding the gel at a constant speed of 7.5 mm/min using a tensile testing machine; and collecting and weighing the last 10 drops of the extruded gel when the displayed force value stabilizes, and calculating the average value of each drop of gel.

In some aspects herein, articles comprising the multi-crosslinked gel system described herein are also provided.

In some embodiments, the multi-crosslinked gel system in the preparation exists in the form of a separate gel system, composition, or composite, such as a composite comprising the multi-crosslinked gel system and particles, microspheres, metals/oxides, fibers, non-woven fabrics, or films composited therewith; a composition comprising the multi-crosslinked gel system and drugs, factors or cells loaded thereon or therein.

In some aspects of the present application, a product is also provided, which comprises the multi-crosslinked gel system or preparations described herein.

In some aspects of the present application, the use of the multi-crosslinked gel system or preparations in the preparation of products for medical treatment, beauty, production, and daily chemical use is also provided.

In some embodiments, the product is selected from the group consisting of vitreous fillers or substitutes, soft tissue or cartilage repairs or fillers, plastic fillers, wound dressings or repairs, drug (e.g., chemical drugs, biological drugs) carriers or drug delivery systems, cell (e.g., stem cells, immune cells) growth or culture carriers or systems, tissue engineering products, and personal care products.

In some embodiments, the product is or comprises a multi-crosslinked gel-microsphere composite, wherein the microsphere includes one or more selected from the group consisting of hydroxyapatite microsphere, polycaprolactone microsphere, polylactic acid microsphere, polyglycolide-lactide microsphere, polyhydroxyalkanoate microsphere, hyaluronic acid microsphere, chitosan microsphere, collagen microsphere, gelatin microsphere, liposome nano-microsphere, and fluorescent microsphere.

In some embodiments, the product is or comprises a multi-crosslinked gel system loaded with cells and/or drugs and/or growth factors, wherein the cells and/or drugs and/or growth factors are directly encapsulated in the gel system or are encapsulated in a microsphere and then uniformly mixed with the gel system. For example, the microsphere includes one or more selected from the group consisting of hydroxyapatite microsphere, polycaprolactone microsphere, polylactic acid microsphere, polyglycolide-lactide microsphere, polyhydroxyalkanoate microsphere, hyaluronic acid microsphere, chitosan microsphere, collagen microsphere, gelatin microsphere, liposome nano-microsphere, and fluorescent microsphere.

In some aspects of the present application, a method for preparing a preparation or product described herein is also provided, which comprises providing the multi-crosslinked gel system described herein; and packaging, combining, and/or compounding the multi-crosslinked gel system.

### Beneficial Effects

The methods and products described herein produce multiple beneficial effects, including:
1. The combination of the long-chain crosslinking agent with the short-chain crosslinking agent or self-crosslinking allows the long-chain crosslinking to initially form a first layer with a loose network, which confers the gel network a certain stability and flexibility and maintains a certain degree of mobility of the polymer; on this basis, the short-chain crosslinking or self-crosslinking forms a second layer with a dense network, which further improves the stability while increasing rigidity and strength.
2. The multi-crosslinked gel system provided by the present invention is formed by combining the long-chain and short-chain crosslinking agents to form double crosslinks, which improves the strength, flexibility, and cohesion of the gel while enhancing its resistance to degradation and prolonging its retention time in the body.
3. The multi-crosslinked gel system provided by the present invention has both good cohesion and stability. It is not easy to diffuse when used for soft tissue filling and exerts a long-term filling effect. Due to the good cohesion, cells or microspheres can be coated so that they do not shift or fall apart in the human body and can remain in the desired parts for a long time. The gel system can also be used to coat growth factors, thereby achieving a long-term sustained release effect.
4. The multi-crosslinked gel system provided by the present invention has good cohesion and stability and a very low swelling rate when used for vitreous filling, and will not cause increased intraocular pressure; it degrades slowly and remains in the body for a long time, enabling long-term vitreous filling; and do not disintegrate into small particles after degradation and does not block the intraocular angle.
5. The multi-crosslinked gel system provided by the present invention has good cohesion and stability and can be used as a surgical dressing in skin wound repair. Its excellent tissue integration makes it resistant to shifting , and it has a sustained release effect when loaded with growth factors or drugs, thereby achieving long-term drug delivery.
6. The method of the present invention is simple and easy to implement and is suitable for scale-up production.

### EXAMPLES AND COMPARATIVE EXAMPLES

The present application will be further described below in conjunction with specific embodiments. It should be understood that these embodiments are only used to illustrate the present application and are not intended to limit the scope of the present application. Those skilled in the art may make appropriate modifications and changes to the present invention, and these modifications and changes are all within the scope of the present invention.

The experimental methods in the following examples where specific conditions are not specified are usually carried out under conventional conditions such as those described in Sambrook et al., Molecular Cloning: A Laboratory Manual (New York: Cold Spring Harbor Laboratory Press, 1989), or under conditions recommended by the manufacturer. Unless otherwise stated, percentages and parts are calculated by weight.

Unless otherwise defined, all professional and scientific terms used herein have the same meanings as those familiar to those skilled in the art. In addition, any methods and materials similar or equivalent to those described herein may be applied to this application. The preferred implementation methods and materials described herein are for demonstration purposes only.

### SOURCES OF MAIN MATERIALS

| **Example** | **Material Name** | Molecular weight (or enzyme activity) | Manufacturer | **Cat. #** |
|---|---|---|---|---|
| 1 | Sodium Hyaluronate (HA) | 1800kDa | Shanghai Likangrui Bioengineering Co., Ltd. | / |
| | Polyethylene glycol diacrylate (PEGDA) | 20kDa | Sangon Biotech (Shanghai) Co., Ltd. | / |
| | Divinyl sulfone (DVS) | 118.15Da | Sigma | 77-77-0 |
| 4 | Carboxymethyl chitosan (CMCS) | 300kDa | Vokai (Beijing Vokai Biotechnology Co., LTD) | 83512-85-0 |
| | Polyglutamic acid | 10kDa | Nanjing Shineking Biotech Co., Ltd | / |
| | DMTMM (coupling agent) | | Suzhou Highfine Biotech Co., Ltd. | 3945-69-5 |
| | Cystamine hydrochloride | 225.19Da | Vokai | 56-17-7 |
| 5 | Sodium Hyaluronate | 600kDa | Bloomage Biotechnology Co., Ltd. | / |
| | Collagen | 300kDa | Shanghai Qi Sheng Biologics Co., Ltd. | / |
| | Polylysine | 4kDa | Zhengzhou Bainafo Bioengineering Co., Ltd. | / |
| | Glutamine transaminase | Enzyme activity: 100U/g | Cangzhou Xiasheng Enzyme Biotechnology Co., Ltd. | 80146-85-6 |
| 6 | Hydroxyapatite microspheres | Particle size: 20-50µm | Synthesized on commission | / |

### EXAMPLE 1. PREPARATION OF DOUBLE CROSSLINKED HYALURONIC ACID GEL

1800 kDa sodium hyaluronate was dissolved in phosphate buffer (initial pH was 6.5, and the pH was adjusted to 13 (using sodium hydroxide as the regulator) to formulate a solution with a mass concentration of 2.5% (W _{hyaluronic acid} /W_{phosphate buffer}). Polyethylene glycol diacrylate (PEGDA, molecular weight of 20 kDa) was added and mixed uniformly, with the molar ratio of PEGDA : sodium hyaluronate monomer being 1:10. The mixture was reacted at 2-8 °C for 48 hours to form a preliminary crosslinked hyaluronic acid network.

The pH was adjusted to 10 and an excess amount of phosphate buffer (pH 7.2) was added to fully disperse and swell the gel. Ethanol (95%) was added, followed by mixing uniformly, and the mixture was stirred at 5°C for 0.5 hours, then at 20°C for 0.5 hours, and at 40 °C for 2 hours. The precipitate was collected and washed repeatedly (the washing solution was 95% ethanol, the same as the solvent for precipitation) It was then redissolved in phosphate buffer solution (pH 7.2) to formulate a 2.5% (W _{hyaluronic acid} / W _{phosphate buffer}) preliminary crosslinked gel.

The pH was adjusted to 13, and divinyl sulfone (DVS) was added. The molar ratio of DVS: hyaluronic acid monomer was 1:5, and the crosslinking temperature was set at 2-8°C. After reacting for 48 hours, the reaction was continued for 18 hours with a 1:2 dilution, followed by another 8 hours with a 1:2 dilution.

The pH was adjusted to 10 and an excess amount of phosphate buffer (pH 7.2) was added to fully disperse and swell the gel. Ethanol was added, followed by mixing uniformly, and the mixture was stirred at 5°C for 1 hour, then at 20°C for 1 hour, and at 40 °C for 2 hours. The precipitate was collected, washed repeatedly, and redissolved in phosphate buffer solution (pH 7.2) to formulate a 2.0% double-crosslinked hyaluronic acid gel. The gel was dialyzed in phosphate buffer solution for 5 days (pH 7.2), and sterilized by moist heat for 15 minutes.

### Performance Testing:

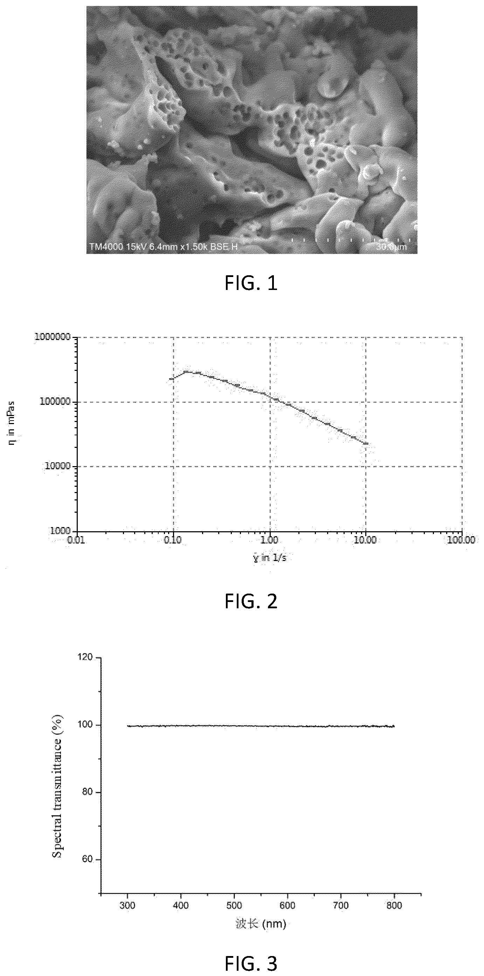

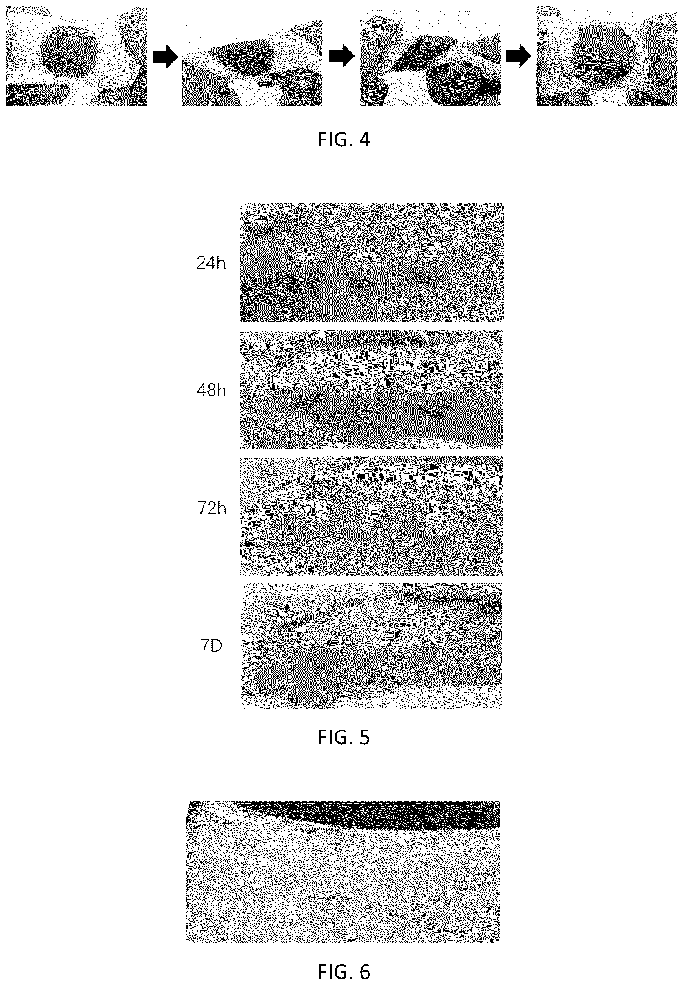

1. The precipitate before the second re-dissolution was vacuum dried; and its morphology was observed using a scanning electron microscope. As shown in FIG. 1, the precipitate exhibited a loose and porous morphology.
2. During the re-dissolution, the precipitate was found to swell completely within 10 minutes, demonstrating a fast dissolution rate.
3. The sterilized double-crosslinked hyaluronic acid gel was tested for its shear viscosity using a rheometer. As shown in FIG. 2, the double-crosslinked hyaluronic acid gel exhibited good viscoelasticity.
4. The transmittance of the double-crosslinked hyaluronic acid gel was tested using a UV spectrophotometer. As shown in FIG. 3, the spectral transmittance of the double-crosslinked hyaluronic acid gel in the wavelength range of 300-800 nm exceeded 98%, indicating an excellent transparency.
5. The refractive index of the double-crosslinked hyaluronic acid gel was tested using a refractometer with a result of 1.334, which is similar to that of natural vitreous (1.3345-1.3348).
6. The double-crosslinked hyaluronic acid gel was dyed and applied to the dermis of pig skin. When folded and twisted arbitrarily, the gel remained firmly attached to the surface of the pig skin (FIG. 4), without shifting or falling apart. This result demonstrated that the double-crosslinked hyaluronic acid gel has excellent cohesion and tissue integration.

### EXAMPLE 2. INTRADERMAL IMPLANTATION TEST OF DOUBLE CROSSLINKED HYALURONIC ACID GEL

The dialyzed and sterilized 2.0% double crosslinked hyaluronic acid gel obtained in Example 1 was injected into the dermis of the back of New Zealand white rabbits, 0.2 mL per implantation point. The skin reaction at the implantation point was observed at 24 h, 48 h, 72 h, and 7 days after implantation. The rabbits were euthanized on day 7, and the gel and surrounding skin tissue were observed intradermally. Samples were collected and sliced for hematoxylin-eosin (HE) staining.

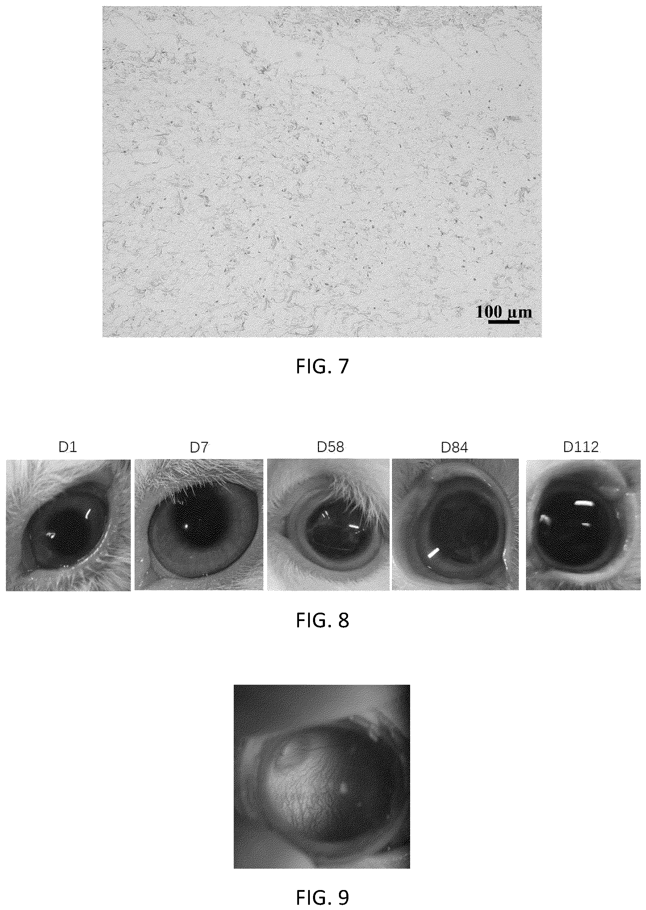

The results showed that no redness, swelling, or eschar was observed on the skin surface or in the inner dermis of the rabbits within 7 days (FIGs 5-6). No inflammatory cell infiltration was observed in the HE staining of the implantation site tissue sections on day 7 (FIG. 7), proving that the double-crosslinked hyaluronic acid gel has good biocompatibility. Compared with common granular tissue fillers that produce a clear interface with skin tissue, the double-crosslinked hyaluronic acid gel penetrated the gaps of the dermis tissue, and the boundary between the gel. The dermis tissue was almost unrecognizable in the photos, proving that the double-crosslinked hyaluronic acid gel has a high degree of fusion with the dermis tissue and excellent tissue integration.

### EXAMPLE 3. EXPERIMENTAL STUDY ON IMPLANTATION OF DOUBLE CROSSLINKED HYALURONIC ACID GEL INTO VITREOUS CAVITY OF ANIMAL EYES

The double crosslinked hyaluronic acid gel obtained in Example 1 was injected into the vitreous cavity of New Zealand white rabbit eyes. The specific test method involved measuring and recording the intraocular pressure of the test rabbits, followed by dilating the pupils with compound tropicamide eye drops. After anesthetizing the animals, the conjunctival sac and periocular hair were disinfected with povidone-iodine, sterile drapes were laid around the surgical area, and the eyelids were opened with an eyelid opener. Three flat scleral openings were made using a 23G vitrectomy puncture knife at a site 1 mm-1.5 mm away from the corneal margin. The perfusion head was placed, the perfusion switch was turned on, and the perfusion tube was fixed with adhesive tape to prevent the perfusion head from damaging the lens. A vitrectomy contact lens was placed on the corneal surface to assist in intraoperative observation. Conventional vitrectomy was performed under a surgical microscope, and triamcinolone acetonide was used to stain the vitreous during the operation. After the posterior pole vitrectomy was completed, gas-liquid exchange was performed, and residual liquid in the posterior pole and optic cup was removed with a flute needle. After vitrectomy, the double crosslinked hyaluronic acid gel was injected to fill the vitreous cavity. The scleral puncture site was closed with absorbable sutures, and tobramycin eye ointment was applied to the conjunctival sac once daily for 3 days postoperatively.

The rabbits were observed regularly and intraocular pressures were measured with an ophthalmotonometer. Fundus oculi images were taken after 16 weeks, and the rabbits were painlessly euthanized. The eyeballs were fixed, sliced, and stained with hematoxylin-eosin to observe the retinal structure.

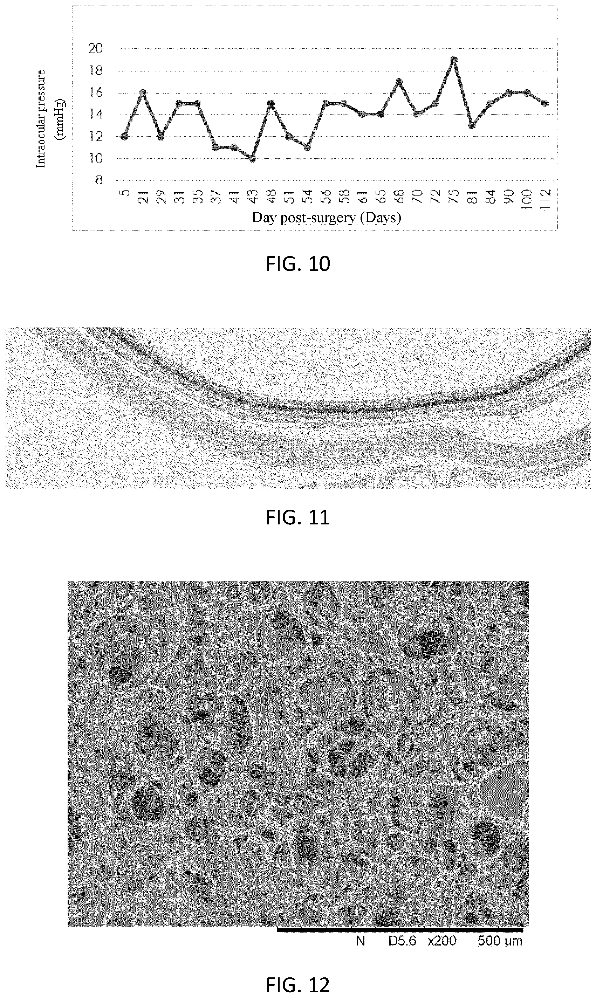

As shown in FIGs 8 and 9, no obvious swelling, inflammation, or whitening of the eyeball was observed during the 16 weeks post-surgery, and no retinal abnormalities were observed at 16 weeks. The test results of the rabbit intraocular pressure (FIG. 10) showed that intraocular pressure was always maintained within the normal range (normal rabbit intraocular pressure is 10-21 mmHg). The retinal staining results after 16 weeks (FIG. 11) showed that the retinal structure was normal and clear, with no signs of obvious degeneration.

The above results demonstrate that the double crosslinked hyaluronic acid gel has good biocompatibility when implanted into the vitreous cavity of the eye, and does not cause increased intraocular pressure, degeneration, or inflammation of surrounding tissues. Additionally, the double crosslinked gel has sufficient viscosity, surface tension and strength, making it suitable for intraocular filling (for example, intraocular filling during retinal surgery) to help opening retinal folds and stabilize the retina. Therefore, the double crosslinked gel of the present application can be used as a vitreous filler in fundus oculi surgeries such as vitrectomy.

### EXAMPLE 4. PREPARATION OF DOUBLE CROSSLINKED CARBOXYMETHYL CHITOSAN GEL

300 kDa carboxymethyl chitosan (CMCS, carboxymethyl substitution degree of 80%) was dissolved in phosphate buffer, and the pH was adjusted to 6.5 to prepare a solution with a mass concentration of 4%. Polyglutamic acid (molecular weight of 10 kDa) and the coupling reagent DMTMM (activated carboxyl group) were added and mixed uniformly. The molar ratio of polyglutamic acid monomer: carboxymethyl chitosan monomer: DMTMM was 1:5:1. The reaction was carried out at 10 °C for 24 hours to form a preliminary crosslinked carboxymethyl chitosan network.

The pH was adjusted to 6.5 and an excess amount of phosphate buffer was added to fully disperse and swell the gel. After adding acetone and mixing uniformly, stirring was conducted at 5 °C for 1 hour, then at 20 °C for 1 hour, and at 40 °C for 2 hours. The precipitate was collected washed repeatedly, and redissolved in phosphate buffer solution to prepare a 3% preliminary crosslinked gel.

The pH was adjusted to 6.5, and cystamine hydrochloride and DMTMM were added. The molar ratio of cystamine: carboxymethyl chitosan monomer: DMTMM was 1:4:2, and the crosslinking temperature was set at 2-8°C. After reacting for 72 hours, the reaction was continued for 18 hours with a 1:2 dilution, and then for another 8 hours with a 1:2 dilution.

The pH was adjusted to 6.5 and an excess amount of phosphate buffer was added to fully disperse and swell the gel. After adding acetone and mixing uniformly, stirring was conducted at 5 °C for 1 hour, then at 20 °C for 1 hour, and at 40 °C for 2 hours. The precipitate was collected, washed repeatedly, and redissolved in phosphate buffer solution to prepare a 2.2% double-crosslinked carboxymethyl chitosan gel. The gel was dialyzed and sterilized by wet heat for 15 minutes.

The double-crosslinked carboxymethyl chitosan gel was freeze-dried, and its morphology was observed using a scanning electron microscope. As shown in FIG. 12, unlike the conventional single crosslinked gel structure, the double crosslinked carboxymethyl chitosan gel exhibits a dense multilayer network structure with both macropores and micropores. The described structure of gel provides advantages such as good permeability, a developed pore structure, a large specific surface area, and good toughness. These properties make it suitable for use as surgical dressings or other applications, facilitating rapid liquid absorption (for example, as a hemostatic or liquid-absorbing material).

### EXAMPLE 5. REPARATION OF DOUBLE CROSSLINKED HYALURONIC ACID-COLLAGEN GEL

600 kDa sodium hyaluronate and 300 kDa collagen were dissolved in a 2% acetic acid solution, and the pH was adjusted to 4.5. The mass concentration of sodium hyaluronate was 1.2%, and the mass concentration of collagen was 4%. Polylysine (molecular weight of 4 kDa) and DMTMM were added and mixed uniformly, and the molar ratio of polylysine: sodium hyaluronate monomer: DMTMM was 1:2:0.6. The reaction was carried out at 2-8 °C for 8 hours to form a preliminary crosslinked hyaluronic acid-collagen network.

The pH was adjusted to 5 and an excess amount of phosphate buffer was added to fully disperse and swell the gel. After adding acetone and mixing uniformly, stirring was conducted at 2°C for 0.5 hours, then at 15°C for 0.5 hours, and at 35°C for 2 hours. The precipitate was collected, washed repeatedly, and redissolved in phosphate buffer solution to prepare a preliminary crosslinked gel with sodium hyaluronate at a mass concentration of 1.2% and collagen at a mass concentration of 4%.

The pH was adjusted to 7, and glutamine and glutamine transaminase were added. The mass concentrations of glutamine and glutamine transaminase were 1% and 0.5%, respectively. The crosslinking temperature was set at 20 °C. After 8 hours of reaction, the reaction was continued for 18 hours with a 1:2 dilution, and then for another 24 hours with a 1:2 dilution.

The pH was adjusted to 5, and an excess amount of phosphate buffer was added to fully disperse and swell the gel. After adding acetone and mixing uniformly, stirring was conducted at 2°C for 0.5 hours, then at 15°C for 0.5 hours, and at 35°C for 2 hours. The precipitate was collected, washed repeatedly, and redissolved in phosphate buffer solution to prepare a double-crosslinked gel with sodium hyaluronate at a mass concentration of 1.2% and collagen at a mass concentration of 4%. The gel was dialyzed.

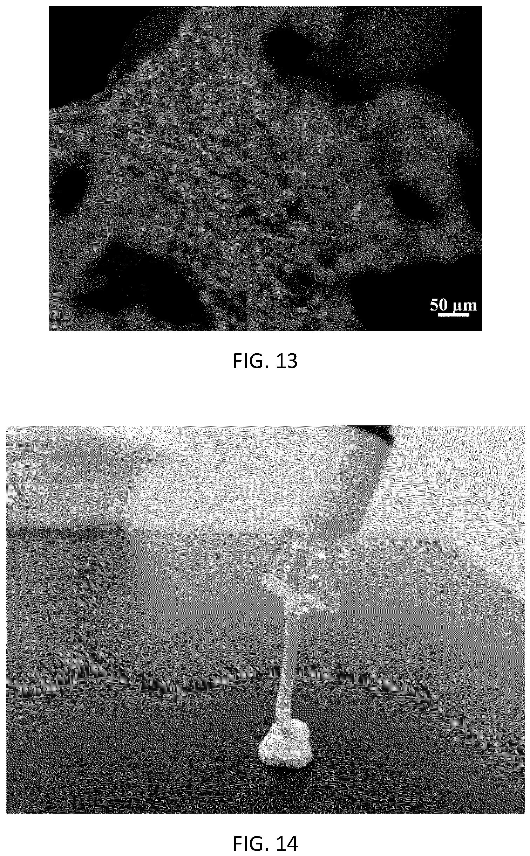

The gel was co-cultured with fibroblasts (purchased from the Cell Bank of the Chinese Academy of Sciences, with a seeding density of 3×10⁴ cells/cm²). The culture conditions were set at 37°C, in a 5% CO₂ incubator, using a high-glucose medium containing 10% fetal bovine serum and 1% double antibodies). The viability of the cells was examined by live-dead cell staining after 7 days of culture. As shown in FIG. 13, the cells grew normally in the gel (the light-colored areas in the figure represent green live cells), and the cells exhibited good viability after 7 days of culture.

### EXAMPLE 6. PREPARATION OF DOUBLE CROSSLINKED CARBOXYMETHYL CHITOSAN GEL-MICROSPHERE COMPOSITE

The double crosslinked carboxymethyl chitosan gel from Example 4 was taken, and hydroxyapatite microspheres were added, with the mass ratio of gel to microspheres being 4:1. A double crosslinked carboxymethyl chitosan gel-microsphere composite was obtained by slowly stirring and mixing.

As shown in FIG. 14, the composite has good viscoelasticity and lifting properties.

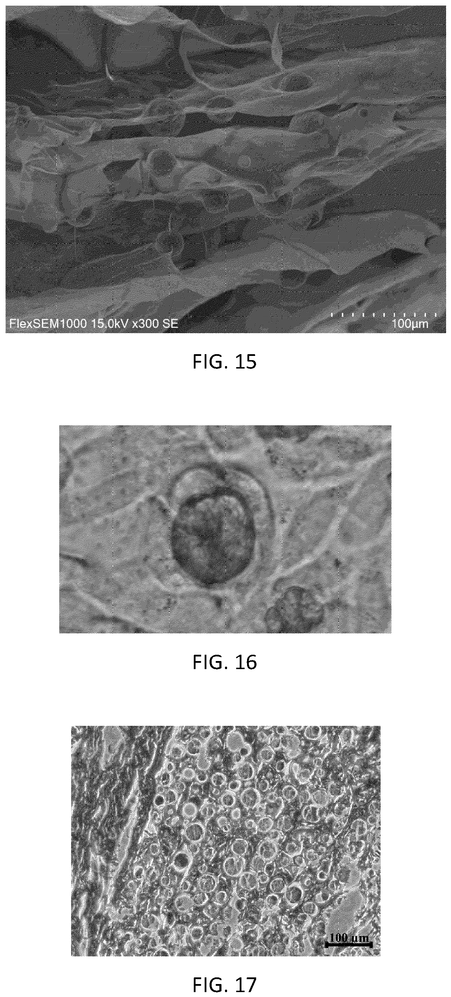

The composite was freeze-dried and its microscopic morphology was observed using a scanning electron microscope. The results showed that the microspheres were embedded in the gel network (FIG. 15). The good cohesion of the gel effectively encapsulated the microspheres in the gel network, making them resistant to diffusion and displacement.

### EXAMPLE 7. CYTOCOMPATIBILITY OF DOUBLE CROSSLINKED CARBOXYMETHYL CHITOSAN GEL-MICROSPHERE COMPOSITE

The double-crosslinked carboxymethyl chitosan gel-microsphere composite from Example 5 was co-cultured with L929 fibroblasts (same method as in Example 5). After 7 days, the cell morphology was observed under an optical microscope. As shown in FIG. 16, the cells adhered to the surface of the microspheres and exhibited a good spreading state.

### EXAMPLE 8. ANIMAL TEST OF DOUBLE CROSSLINKED CARBOXYMETHYL CHITOSAN GEL-MICROSPHERE COMPOSITE AS SOFT TISSUE FILLER

The double crosslinked carboxymethyl chitosan gel-microsphere composite from Example 6 was injected into the deep dermis of the back of New Zealand white rabbits, 0.5 mL at each implantation point. After conventional feeding for one (1) month, the gel and surrounding skin tissue samples were collected, sliced, and stained with Masson's trichrome and Sirius red staining, respectively.

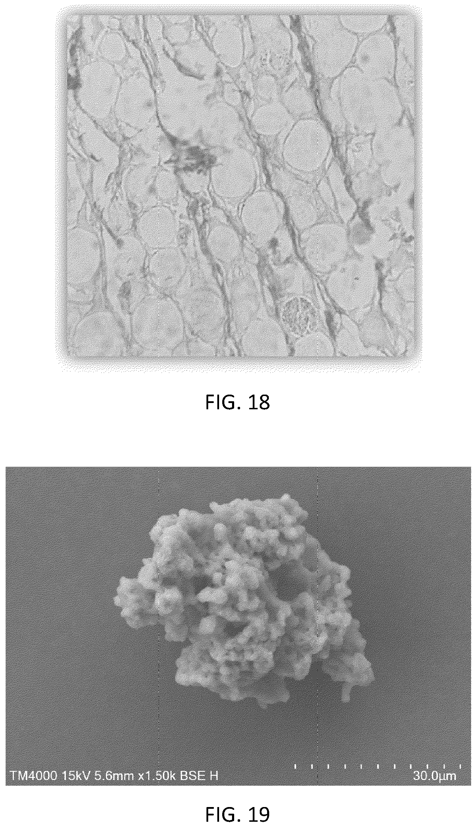

As shown in FIG. 17, one month after the composite was implanted into the dermis, collagen fibers were partially generated. The collagen fibers were distributed in the gel gaps and on the surface of the microspheres. Sirius red staining (FIG. 18) showed that type I and type III collagen were arranged in parallel between the microsphere gaps.

The above results demonstrate that the double-crosslinked carboxymethyl chitosan gel-microsphere composite can be used as a soft tissue filler for dermal implantation, with good biocompatibility and the ability to promote collagen regeneration.

### COMPARATIVE EXAMPLE 1. PREPARATION OF HYALURONIC ACID GEL CROSSLINKED BY SHORT-CHAIN CROSSLINKING AGENT

1800 kDa sodium hyaluronate was dissolved in phosphate buffer, and the pH was adjusted to 13 to prepare a solution with a mass concentration of 2.5%. DVS was added, with the molar ratio of DVS : hyaluronic acid monomer being 1:4, and the reaction was conducted at 5-15 °C for 48 hours. In the present comparative example, to enhance the crosslinking effect of the single crosslinking agent, the amount of crosslinking agent DVS relative to the hyaluronic acid monomer was increased (from DVS: HA=1:5 in Example 1 to 1:4). An excess amount of phosphate buffer was added to fully disperse and swell the gel. Then, ethanol was added, and the mixture was uniformly stirred at room temperature for 3 hours. The precipitate was collected, washed repeatedly, and redissolved in phosphate buffer solution to prepare a 2.0% single crosslinked gel. The gel was dialyzed and sterilized by wet heat for 15 minutes.

### COMPARATIVE EXAMPLE 2. PREPARATION OF HYALURONIC ACID GEL WITH SINGLE CROSSLINKING BY LONG-CHAIN CROSSLINKING AGENT

1800 kDa sodium hyaluronate was dissolved in phosphate buffer, and the pH was adjusted to 13 to formulate a solution with a mass concentration of 2.5%. PEGDA (molecular weight 20 kDa) was added, with the molar ratio of PEGDA: hyaluronic acid monomer being 1:5, and the reaction was conducted at 2-8 °C for 48 hours. In the present comparative example, to enhance the crosslinking effect of the single crosslinking agent, the amount of the crosslinking agent PEGDA relative to the hyaluronic acid monomer was increased (from PEGDA: HA=1:10 in Example 1 to 1:5). An excess amount of phosphate buffer was added to fully disperse and swell the gel. Then, ethanol was added and the mixture was uniformly stirred at room temperature for 3 hours. The precipitate was collected, washed repeatedly, and redissolved in phosphate buffer solution to prepare a 2.0% single crosslinked gel. The gel was dialyzed and sterilized by wet heat for 15 minutes.

### COMPARATIVE EXAMPLE 3. PREPARATION OF SINGLE CROSSLINKED HYALURONIC ACID MIXED GEL

The sterilized single-crosslinked sodium hyaluronate gels from Comparative Examples 1 and 2 were mixed uniformly in equal weight to form a physical mixed gel of single-crosslinked DVS and single-crosslinked PEGDA.

### COMPARATIVE EXAMPLE 4. COMPARISON OF THE COHESION BETWEEN DOUBLE-CROSSLINKED HYALURONIC ACID GEL AND SINGLE-CROSSLINKED HYALURONIC ACID GEL

The gels from Example 1 and Comparative Examples 2-4 were tested for cohesion using the cohesion test method disclosed in Dermatol Surg 2015; 41: S365-S372.

The sample was loaded into a 1 mL glass syringe and centrifuged (at 2500-3000 rpm) for 5-10 minutes to remove the air in the gel. An 18G needle (inner diameter of 0.9 mm) and a plunger were configured on the syringe. The gel was extruded at a constant speed of 7.5 mm/min using a tensile testing machine. When the displayed force value stabilized, the last 10 drops of extruded gel were collected and weighed, and the average value of each drop of gel was calculated.

According to this method, the greater the gel mass, the stronger the cohesion. The test results are shown in the following table:

| **Sample grouping** | **Average gel mass per drop (mg)** |
|---|---|
| Example 1 (long and short chain crosslinking agents, double crosslinking) | 65 |
| Comparative Example 2 (short chain crosslinking agent, single crosslinking) | 32 |
| Comparative Example 3 (long-chain crosslinking agent, single crosslinking) | 38 |
| Comparative Example 4 (single crosslinked gel, physical mixing) | 25 |

From the above results, it can be seen that the cohesion of the double-crosslinked sodium hyaluronate gel is significantly better than that of the single-crosslinked gel, proving that the combination of long-chain and short-chain crosslinking agents to crosslink the polymer gel helps to improve the cohesion of the gel. Meanwhile, the mixed gel prepared by crosslinking sodium hyaluronate with the long-chain crosslinking agent and the short-chain crosslinking agent respectively, and then physically mixing has a lower cohesion compared with the single-crosslinked and double-crosslinked gels. This may be because the long-chain crosslinking agent and the short-chain crosslinking agent are respectively single crosslinked to form different phases, and after physical mixing, the hydrogen bonds in the original phases are broken, forming an immiscible double phase, resulting in a decrease in the intramolecular force of the overall gel.

### COMPARATIVE EXAMPLE 5. PREPARATION AND COMPARISON OF DOUBLE CROSSLINKED HYALURONIC ACID GEL

In the present comparative example, a double crosslinked hyaluronic acid gel was prepared by the same method as in Example 1, but no gradient heating was applied during the poor solvent replacement process after the first and second crosslinking. After the short-chain crosslinking agent was crosslinked, the pH was adjusted to 10, and an excess amount of phosphate buffer was added to fully disperse and swell the gel. Then, ethanol was added and the mixture was stirred at room temperature for 4 hours. The precipitate was taken, repeatedly washed, and redissolved in a phosphate buffer solution to formulate a 2.0% double crosslinked hyaluronic acid gel.

The precipitate before the re-dissolution was taken and vacuum dried, and the morphology of the precipitate was observed using a scanning electron microscope.

As shown in FIG. 19, the precipitate obtained without applying gradient heating during the poor solvent replacement process is an agglomeration of a plurality of small particles, which is different from the porous morphology presented in Example 1.

When the precipitate was redissolved, it was found that it took 16 hours for the precipitate to completely swell, and the dissolution rate was significantly lower than the result in Example 1.

All references cited herein are incorporated by reference in their entirety as though each reference was individually incorporated by reference. It should be understood that after reading the contents of this application, those skilled in the art may conduct various changes or modifications to the present application, and these equivalent forms also fall within the scope defined by the claims attached to this application.

## Claims

1. A method for preparing a multi-crosslinked gel system comprising the following steps:
(1) subjecting the high-molecular compound to a first crosslinking reaction in the presence of a long-chain crosslinking agent under a condition suitable for crosslinking to obtain a preliminarily crosslinked gel network, wherein the long-chain crosslinking agent has a molecular weight of no less than 400 Da and/or a chain length of no less than 12 backbone skeleton atoms;
(2) swelling, precipitating, and redissolving the preliminarily crosslinked gel network to form a preliminarily crosslinked gel;
(3) subjecting the preliminarily crosslinked gel to a second crosslinking reaction in the presence of a short-chain crosslinking agent and/or a condensing agent under a condition suitable for crosslinking to obtain a secondary crosslinked gel network, wherein the short-chain crosslinking agent has a molecular weight of less than 400 Da and/or a chain length of less than 12 backbone skeleton atoms;
(4) swelling, precipitating, and redissolving the secondary crosslinked gel network to form a multi-crosslinked gel system.

2. The method according to claim 1, wherein the molecular weight of the high-molecular compound is no less than 10,000 Da, and the side chain comprises two or more carboxyl groups or amino groups or hydroxyl groups or a combination thereof,
for example, the high-molecular compound is one or more selected from the group consisting of hyaluronic acid (such as sodium hyaluronate), chitosan, carboxymethyl chitosan, chitin, carboxymethyl chitin, collagen, gelatin, silk fibroin, carboxymethyl dextran, carboxymethyl cellulose, amino polyethylene glycol, and carboxyl polyethylene glycol; and/or
the initial concentration of the high-molecular compound in the aqueous solution is 0.2% to 10%, such as 0.4% to 8%.

3. The method according to claim 1, wherein the long-chain crosslinking agent is selected from the group consisting of amino polyethylene glycol, carboxyl polyethylene glycol, polyethylene glycol diglycidyl ether (PEGDE), polyglutamic acid, polylysine, polyaspartic acid and their salts, tannic acid, proanthocyanidins, and has a molecular weight of no less than 400 Da and/or a chain length of no less than 12 backbone skeleton atoms; and/or
the molar ratio of the long-chain crosslinking agent to the crosslinking reactive group in the high-molecular compound is 1:10 to 1:2.

4. The method according to claim 1, wherein the short chain crosslinking agent is selected from the group consisting of glutamic acid, lysine, aspartic acid and their salts and polymers, glutamine, divinyl sulfone (DVS), butanediol diglycidyl ether (BDDE), polyethylene glycol diglycidyl ether (PEGDE), diepoxyoctane (DEO), cystamine and the salt thereof, hexamethylenediamine, p-phenylene carbodiimide (BCDI), amino polyethylene glycol, carboxyl polyethylene glycol, glutaraldehyde, genipin, and has a molecular weight of less than 400 Da and/or a chain length of less than 12 backbone skeleton atoms; and/or
the molar ratio of the short-chain crosslinking agent to the crosslinking reactive group in the high-molecular compound is 1:10 to 1:1.

5. The method according to claim 1, wherein step (1) and/or step (3) may optionally further comprise adding a condensing agent to the crosslinking reaction system,
for example, the condensing agent used is selected from the group consisting of 1-ethyl-3-(3-dimethyl aminopropyl) carbodiimide hydrochloride (EDC), 1,3-dicyclohexylcarbodiimide (DCC), glutamine aminotransferase, 4-(4,6-dimethoxy triazine-2-yl)-4-methyl morpholine hydrochloride (DMTMM), 6-chloro-2,4-dimethoxy-1,3,5-triazine (CDMT); and/or
the molar ratio of the reactive groups of the condensing agent to the crosslinking agent in the reaction system comprising the condensing agent is 1:5 to 2:1; and/or
when the molecular chain of the high-molecular compound has both carboxyl and amino reactive groups, the condensing agent in the crosslinking reaction system causes the molecules to self-crosslink.

6. The method according to claim 1, wherein step (1) further comprises providing an aqueous solution of the high-molecular compound (e.g., a phosphate buffer solution, an aqueous solution), and adjusting its pH to be suitable for the first crosslinking reaction, for example:
the aqueous solution is selected from phosphate buffer solution, acetic acid solution, aqueous solution; and/or
the mass percentage concentration of the high-molecular compound in the solution is 0.4% to 8%; and/or
the reaction temperature of the first crosslinking reaction is 0°C to room temperature, for example, 2°C to 30°C; and/or
the reaction time of the first crosslinking reaction is 5 to 120 hours, for example, 8 to 100 hours.

7. The method according to claim 1, wherein step (2) and step (4) each independently comprise one or more selected from the group consisting of:
(a) adjusting the pH of the gel network obtained in the previous step, for example:
when the high-molecular compound with the highest concentration in the system is hyaluronic acid, silk fibroin, carboxymethyl dextran, carboxymethyl cellulose, and/or carboxy polyethylene glycol, the pH value is adjusted to 7.5 to 14;
when the high-molecular compound with the highest concentration in the system is chitosan, carboxymethyl chitosan, chitin, carboxymethyl chitin, collagen, gelatin, and/or amino polyethylene glycol, the pH value is adjusted to 2 to 6.5;
(b) the swelling comprises: fully swelling and equilibrating the obtained gel network in an aqueous solution;
(c) the precipitating comprises: adding a poor solvent to an aqueous solution comprising the fully swollen gel network and mixing to precipitate the gel network, for example:
gradually heating the mixture during the precipitation process, for example, from 0°C to 50°C, such as 2 to 5°C for 0.5 to 2 hours, 15 to 25°C for 0.5 to 2 hours, and 35 to 45°C for 1 to 4 hours;
the poor solvent is selected from acetone, ethanol, chloroform, ether, n-hexane, or a combination thereof;
(d) optionally, washing the resulting precipitate one or more times; and/or
(e) the re-dissolving comprises: re-dissolving part or all of the precipitate in an aqueous solution, for example, to obtain a mass percentage concentration of 1 to 10% (such as 1.5-8%) gel solution.

8. The method according to claim 1, wherein step (3) comprises:
adjusting the pH of the preliminary crosslinked gel obtained in the previous step; and/or
performing a gradient dilution during the crosslinking reaction, for example, 1 to 5 times dilution after 8 to 72 hours of reaction, followed by 1 to 5 times dilution after 8 to 48 hours of reaction, and then 8 to 24 hours of reaction; and/or
the reaction temperature of the second crosslinking reaction is 0°C to room temperature, for example, 2°C to 30°C; and/or
the reaction time of the second crosslinking reaction is 5 to 120 hours, for example, 8 to 100 hours.

9. The method according to claim 1, wherein the method further comprises purifying and/or sterilizing the obtained multi-crosslinked gel system; and/or
the modification degree of the long-chain crosslinking agent in the multi-crosslinked gel system is 2% to 40%, and the modification degree of the short-chain crosslinking agent or self-crosslinking is 5% to 40%.

10. The method according to claim 1 comprises the following steps:
(1') preparing an aqueous solution of a high-molecular compound, adjusting the pH, adding a long-chain crosslinking agent, and performing a first crosslinking reaction with or without the presence of a condensing agent to obtain a preliminarily crosslinked gel network;
(2') adjusting the pH, and then placing the gel network in step (1') in an aqueous solution to fully swell and balance, adding a poor solvent, mixing uniformly and then gradually increasing the temperature, taking the precipitate and repeatedly washing, redissolving it in an aqueous solution, and formulating a preliminary crosslinked gel;
(3') adjusting the pH, adding a short-chain crosslinking agent and/or a condensing agent to the preliminarily crosslinked gel to carry out a second crosslinking reaction, and performing a gradient dilution during the reaction;
(4') taking the gel obtained in step (3'), adjusting the pH, and then placing the gel network in step (3') in an aqueous solution to fully swell and balance, adding a poor solvent, mixing uniformly, and then gradually heating, taking the precipitate and repeatedly washing, and redissolving it in an aqueous solution to obtain a multi-crosslinked gel network;
(5') optionally, the multi-crosslinked gel network is purified, sterilized, and/or packaged.

11. The method according to any one of claims 1 to 10, further comprising selecting the length of the long-chain crosslinking agent and the short-chain crosslinking agent and/or the amount ratio of the long-chain crosslinking agent to the short-chain crosslinking agent to control the crosslinking modification degree of the multi-crosslinked gel network.

12. A multi-crosslinked gel system, wherein the multi-crosslinked gel system is prepared by the method according to any one of claims 1 to 11.

13. A multi-crosslinked gel system, wherein the multi-crosslinked gel system comprises:
a first layer network obtained by crosslinking using a long-chain crosslinking agent; and
a second layer network obtained by using short-chain crosslinking agents or self-crosslinking,
wherein the density of the first layer network is lower than that of the second layer network.

14. The multi-crosslinked gel system according to claim 12 or 13, wherein the multi-crosslinked gel system has a cohesiveness of an average mass of each gel drop of no less than 40 mg as measured by:
loading the sample into a 1 mL glass syringe, and centrifuging at 2500-3000 rpm for 5-10 minutes to remove the air in the gel; installing an 18G needle with an inner diameter of 0.9 mm and a plunger on the syringe; extruding the gel at a constant speed of 7.5 mm/min using a tensile testing machine; and collecting and weighing the last 10 drops of the extruded gel when the displayed force value stabilized, and calculating the average value of each drop of gel.

15. A preparation comprising the multi-crosslinked gel system according to any one of claims 12 to 14.

16. The preparation according to claim 15, wherein the multi-crosslinked gel system in the preparation exists in the form of a separate gel system, composition, or composite, such as a composite comprising the multi-crosslinked gel system and particles, microspheres, metals/oxides, fibers, non-woven fabrics, or films composited therewith; or a composition comprising the multi-crosslinked gel system and drugs, factors or cells loaded thereon or therein.

17. A product comprising the multi-crosslinked gel system according to any one of claims 12 to 14 or the preparation according to claim 15 or 16.

18. Use of the multi-crosslinked gel system according to any one of claims 12 to 14 or the product according to claim 15 or 16 in the preparation of medical, cosmetic, production, and daily chemical products.

19. The product according to claim 17 or the use according to claim 18, wherein the product is selected from the group consisting of vitreous fillers or substitutes, soft tissue or cartilage repairs or fillers, plastic fillers, wound dressings or repairs, drug (e.g., chemical drugs, biological drugs) carriers or drug delivery systems, cell (e.g., stem cells, immune cells) growth or culture carriers or systems, tissue engineering products, and personal care products; and/or
the product is or comprises a multi-crosslinked gel-microsphere composite, wherein the microsphere includes one or more selected from the group consisting of hydroxyapatite microspheres, polycaprolactone microspheres, polylactic acid microspheres, polyglycolide-lactide microspheres, polyhydroxyalkanoate microspheres, hyaluronic acid microspheres, chitosan microspheres, collagen microspheres, gelatin microspheres, liposome nano-microspheres, and fluorescent microspheres; and/or
the product is or comprises a multi-crosslinked gel system loaded with cells and/or drugs and/or growth factors, wherein the cells and/or drugs and/or growth factors are directly encapsulated in the gel system or are encapsulated in microspheres and then uniformly mixed with the gel system; for example, the microspheres include one or more selected from the group consisting of hydroxyapatite microspheres, polycaprolactone microspheres, polylactic acid microspheres, polyglycolide-lactide microspheres, polyhydroxyalkanoate microspheres, hyaluronic acid microspheres, chitosan microspheres, collagen microspheres, gelatin microspheres, liposome nano-microspheres, and fluorescent microspheres.

20. A method for preparing the preparation according to claim 15 or 16, comprising providing the multi-crosslinked gel system according to any one of claims 12 to 14; and packaging, combining and/or compounding the multi-crosslinked gel system.
